# FASCICULE DE BREVET EUROPEEN

(11) **EP 2 331 190 B1**
(45) Date de publication et mention de la délivrance du brevet: **17.10.2012**
(21) Numéro de dépôt: 09797578.3
(22) Date de dépôt: 07.07.2009
(51) Int. Cl.: A61M 37/00, A61M 5/142, A61M 5/158

(54) **DISPOSITIF D'INJECTION MINIATURISE A USAGE MEDICAL A CARTOUCHE AMOVIBLE**
MINIATURISIERTES INJEKTIONSGERÄT ZUR MEDIZINISCHEN ANWENDUNG MIT EINER HERAUSNEHMBAREN PATRONE
MINIATURIZED INJECTION DEVICE FOR MEDICAL USE COMPRISING A REMOVABLE CARTRIDGE

(30) Priorité: 18.07.2008 FR 0854920
(43) Date de publication de la demande: 15.06.2011
(73) Titulaire: Eveon, 38330 Montbonnot Saint Martin (FR)
(72) Inventeur: PERRIERE, Bernard, F-38130 Echirolles (FR)
(74) Mandataire: Prugneau, Philippe
(86) Numéro de dépôt international: PCT/FR2009/051345
(87) Numéro de publication internationale: WO 2010/007296

(56) Documents cités:
- WO-A-2008/101892
- FR-A- 2 909 001
- US-A- 4 886 499
- US-A1- 2004 134 495
- US-A1- 2006 264 926

## Description

L'invention concerne un dispositif d'injection miniaturisé à usage médical apte à réaliser l'injection d'une substance médicamenteuse par voie sous cutanée ou intramusculaire chez un être humain ou chez un animal.

L'invention concerne plus particulièrement un dispositif d'injection miniaturisé à usage médical comprenant un boîtier formant une enceinte allongée selon une direction axiale destinée à recevoir une cartouche définissant un réservoir pour une substance médicamenteuse à injecter, et comprenant en outre au moins une aiguille destinée à être raccordée au réservoir quand la cartouche est insérée dans l'enceinte du boîtier, et une pompe montée avec l'aiguille sur un support mobile en translation selon ladite direction axiale dans l'enceinte, la pompe étant agencée pour faire circuler la substance médicamenteuse à injecter depuis le réservoir vers l'aiguille.

En particulier, l'invention vise un dispositif d'injection à usage médical apte à permettre qu'une injection, qu'elle soit hypodermique, intradermique, intramusculaire ou intraveineuse, soit effectuée facilement et sans appréhension à un patient seul ou suivi médicalement. De manière générale, de tels dispositifs d'injection se présentent sous la forme de seringues, par exemple des stylos seringues, tenues en main par un utilisateur.

Les dispositifs d'injection ont généralement une aiguille visible et sont de dimensions suffisantes pour permettre à l'utilisateur de réaliser la visée d'une zone d'injection visible à l'oeil. Or, la simple vision de l'aiguille peut stresser certains utilisateurs pour lesquels une injection avec un tel dispositif peut être psychologiquement difficile, voir traumatisant. Pour certaines personnes, il est même impossible de procéder à une auto-injection, ce qui peut avoir des conséquences dramatiques dans le cas de patients isolés, par exemple pour des patients souffrant de diabète nécessitant des injections régulières d'insuline ou lors de crises imprévisibles.

De plus, il peut arriver qu'un utilisateur non formé doive pratiquer une injection sur un patient, dans le cas d'une intervention d'urgence par exemple. Il existe alors, en plus de l'appréhension éventuelle, un risque pour l'utilisateur de se blesser avec l'aiguille après l'injection, voire de se contaminer si le patient présente une maladie infectieuse. Par ailleurs, un utilisateur peut être amené à décider au dernier moment de la substance médicamenteuse à injecter en fonction de la pathologie du patient.

Il existe donc un besoin pour un dispositif d'injection de substance médicamenteuse qui soit suffisamment simple d'utilisation à la fois pour qu'un patient puisse réaliser une auto-injection et pour qu'un utilisateur non formé puisse pratiquer une injection en toute sécurité et sans aucune appréhension.

On connaît du document FR2909001 un dispositif médical d'injection tel que décrit ci-dessus. Cependant, dans ce dispositif, il n'est pas prévu de réservoir amovible que l'utilisateur peut insérer facilement dans la cartouche.

On connaît du document US-5928205 un dispositif médical d'injection constitué d'un tube dans lequel une cartouche est insérée, la cartouche comprenant un réservoir et une aiguille séparée du réservoir par une membrane dans un état initial du dispositif et protégée de l'environnement extérieur par un couvercle. Pour faire une injection, l'utilisateur manipule d'abord le couvercle de sorte que l'aiguille perce la membrane, puis il retire le couvercle. Ce dispositif permet donc de masquer l'aiguille avant utilisation, mais présente l'inconvénient que l'aiguille reste visible par l'utilisateur d'une part pendant l'injection et d'autre part en fin d'injection ce qui implique un risque de blessure ou de contamination accidentelle. En outre, ce dispositif nécessite l'utilisation de cartouches spécifiques ayant une paroi mobile de type piston.

On connaît aussi du document WO2006/062788 un dispositif médical d'injection comprenant un tube contenant une cartouche de produit à injecter ayant une paroi d'extrémité mobile et équipée d'une aiguille, le tube étant muni à une extrémité d'une ouverture pour laisser passer l'aiguille. Le dispositif comprend un moyen d'entrainement permettant à la fois d'entrainer l'aiguille à travers l'ouverture et de faire coulisser la paroi mobile de la cartouche. Le dispositif peut être réutilisé pour une seconde injection. Cependant, l'aiguille n'est pas rétractable entre les deux injections, mais seulement protégée par un couvercle, ce qui n'empêche pas les risques de blessure ou de contamination accidentelle. De plus, ce dispositif nécessite l'utilisation de cartouches spécifiques ayant une paroi mobile et munies d'une aiguille, que l'utilisateur doit monter/démonter dans le tube selon une procédure particulière.

On connait également du document US2006-264926 un dispositif d'injection miniaturisé à usage médical comprenant un boîtier formant une enceinte dans laquelle est logée une cartouche définissant un réservoir souple pour une substance médicamenteuse à injecter. La cartouche est portée par une plaque principale mobile en translation dans l'enceinte. Cette plaque principale est couplée à une plaque secondaire de manière à définir entre-elles une chambre secondaire étanche. La périphérie de la plaque principale est reliée de manière étanche à la paroi interne de l'enceinte de sorte à partager l'enceinte, de manière étanche, en une chambre inférieure étanche et une chambre supérieure reliée à l'air libre. La plaque principale est traversée par un orifice principal reliant la chambre inférieure à l'intérieur du réservoir souple prévu dans la chambre secondaire. Avant utilisation, cet orifice est obturé par une membrane. La plaque principale est également traversée par des orifices secondaires. Le dispositif d'injection comprend en outre une aiguille portée par un bloc mobile en translation dans l'enceinte de sorte que, avant utilisation, les deux extrémités de l'aiguille sont libres. Ce bloc est séparé de la plaque principale par un ressort évitant que l'extrémité libre supérieure de l'aiguille ne transperce la membrane. L'embase de l'enceinte destinée à être placée au dessus du site d'injection est pourvue d'une cavité. Cette embase est par ailleurs traversée par des orifices reliant la cavité à la chambre inférieure. Ce dispositif d'injection comporte en outre des moyens d'aspiration externes à l'enceinte aptes, lorsque le dispositif d'injection est appliqué contre la peau, à aspirer la peau dans la cavité et à générer une dépression dans la chambre inférieure. Cette dépression provoque le déplacement de la plaque principale vers l'embase et donc, dans un premier temps, le déplacement de l'aiguille vers l'embase. L'aiguille traverse ainsi la peau soulevée par l'aspiration. Dans un second temps, une fois le bloc portant l'aiguille en butée dans l'embase, l'aspiration est prolongée de sorte que le déplacement de la plaque principale vers l'embase provoque la compression du ressort suivi du transpercement de la membrane par l'extrémité de l'aiguille traversant l'orifice principal. Dans un troisième temps, une fois le ressort comprimé, la dépression est prolongée de sorte à provoquer le rapprochement de la plaque secondaire vers la plaque principale, l'écrasement du réservoir souple et l'expulsion de la substance médicamenteuse au travers de l'aiguille. Ce dispositif d'injection nécessite l'utilisation de moyens d'aspiration externes.

Le but de la présente invention est donc de proposer un dispositif d'injection modulaire en ce qu'il comporte un boîtier apte à recevoir une cartouche choisie parmi des cartouches pré-remplies de différentes substances médicamenteuses à injecter.

Un autre but de l'invention est de proposer un dispositif d'injection qui permette d'entreposer des cartouches séparément des boîtiers, puis d'assembler facilement et simplement une cartouche sur un boîtier juste avant de procéder à une injection tout en respectant un maximum d'hygiène, c'est-à-dire en interdisant une réutilisation du boîtier avec une autre cartouche en dehors d'une phase de recyclage.

A cet effet, l'invention a pour objet un dispositif d'injection miniaturisé à usage médical comprenant un boîtier formant une enceinte allongée selon une direction axiale destinée à recevoir une cartouche définissant un réservoir pour une substance médicamenteuse à injecter, et comprenant en outre au moins une aiguille destinée à être raccordée au réservoir quand la cartouche est insérée dans l'enceinte du boîtier, et une pompe montée avec l'aiguille sur un support mobile en translation selon ladite direction axiale dans l'enceinte, la pompe étant agencée pour faire circuler la substance médicamenteuse à injecter depuis le réservoir vers l'aiguille, caractérisé en ce qu'il comprend en outre :
- une première partie d'un système de fixation du type mâle/femelle à crans de verrouillage pour fixer la cartouche dans l'enceinte qui est destinée à s'accoupler de manière irréversible par insertion selon ladite direction axiale avec une deuxième partie du système de fixation prévue sur la cartouche quand la cartouche est suffisamment insérée dans l'enceinte selon ladite direction axiale,
- un moyen de sécurité agencé pour empêcher le déplacement du support mobile selon ladite direction axiale lorsque la cartouche est insérée dans l'enceinte et
- un moyen interrupteur qui est actionné quand le support mobile a été suffisamment déplacé selon ladite direction axiale pour commander alors le fonctionnement de la pompe.

Le boîtier et la cartouche du dispositif d'injection miniaturisé à usage médical selon l'invention sont destinés à être conditionnés séparément l'un de l'autre avant utilisation et après une utilisation, c'est-à-dire après une injection, l'ensemble est destiné à être recyclé. Le concept du dispositif d'injection sans piston à cartouche interchangeable selon l'invention est donc d'intégrer facilement dans un boîtier standard des flacons pouvant avoir des capacité différentes et ce pour un usage unique du boîtier avec un flacon.

Le dispositif d'injection selon l'invention est particulièrement bien adapté à un usage unique pour des injections de vaccins ou de traitement d'urgence puisque l'on peut emmener des boîtiers stériles et plusieurs cartouches stériles contenant des vaccins ou substances médicamenteuses différentes et choisir la substance à injecter juste avant l'injection.

Le dispositif d'injection selon l'invention peut présenter les particularités suivantes :
- les première et deuxième parties du système de fixation sont agencées pour réaliser un guidage axial de la cartouche dans l'enceinte quand la cartouche est insérée dans l'enceinte selon ladite direction axiale ;
- le dispositif d'injection comprend au moins un cône de percussion qui est fixé au support et qui est raccordé à la pompe, les crans de verrouillage des première et seconde parties du système de fixation étant agencés pour se verrouiller quand le cône pénètre dans le réservoir de la cartouche ;
- le dispositif d'injection comprend en outre un moyen de blocage agencé pour laisser passer une seule fois dans un sens et dans l'autre le support selon ladite direction axiale et pour l'empêcher de passer une seconde fois dans le même sens selon cette direction axiale ;
- une première partie femelle du système de fixation est prévue dans un trou borgne aménagé à l'intérieur de l'enceinte du boîtier et une deuxième partie mâle du système de fixation est prévue sur la cartouche à une extrémité d'un bras s'étendant selon ladite direction axiale et s'insérant dans le trou borgne ;
- la partie mâle du système de fixation est prévue sur un couvercle de la cartouche à des extrémités respectives de deux bras diamétralement opposés par rapport au couvercle de la cartouche et s'insérant dans deux trous borgnes diamétralement opposés aménagés à l'intérieur de l'enceinte ;
- le boîtier comprend un premier tube coulissant dans un deuxième tube selon la direction axiale, la pompe avec l'aiguille et la cartouche étant solidaires en mouvement avec le premier tube selon ladite direction axiale et le premier moyen de blocage est une bague de sécurité maintenue entre deux collerettes bordant respectivement le premier et le second tube ;
- le boîtier comprend un premier tube coulissant dans un deuxième tube selon la direction axiale, la pompe avec l'aiguille et la cartouche étant solidaires en mouvement avec le premier tube selon la direction axiale et le second moyen est un interrupteur qui est monté sur le support et qui est actionné par un pion disposé sur le deuxième tube ;
- le couvercle de la cartouche est muni d'un évidemment dans lequel s'emboîte le réservoir.

Un exemple d'un mode de réalisation particulier du dispositif d'injection selon l'invention est décrit plus en détail ci-après et illustré par les dessins. Cette description n'est donnée qu'à titre d'exemple indicatif et nullement limitatif de l'invention.
La figure 1 est une vue schématique en coupe d'un dispositif d'injection selon l'invention montrant une enceinte et une cartouche.
La figure 2 est une vue schématique en perspective du dispositif d'injection de la figure 1 avant insertion de la cartouche dans l'enceinte.
La figure 3 est une vue schématique en perspective du dispositif d'injection de la figure 1 une fois que la cartouche a été insérée dans l'enceinte.

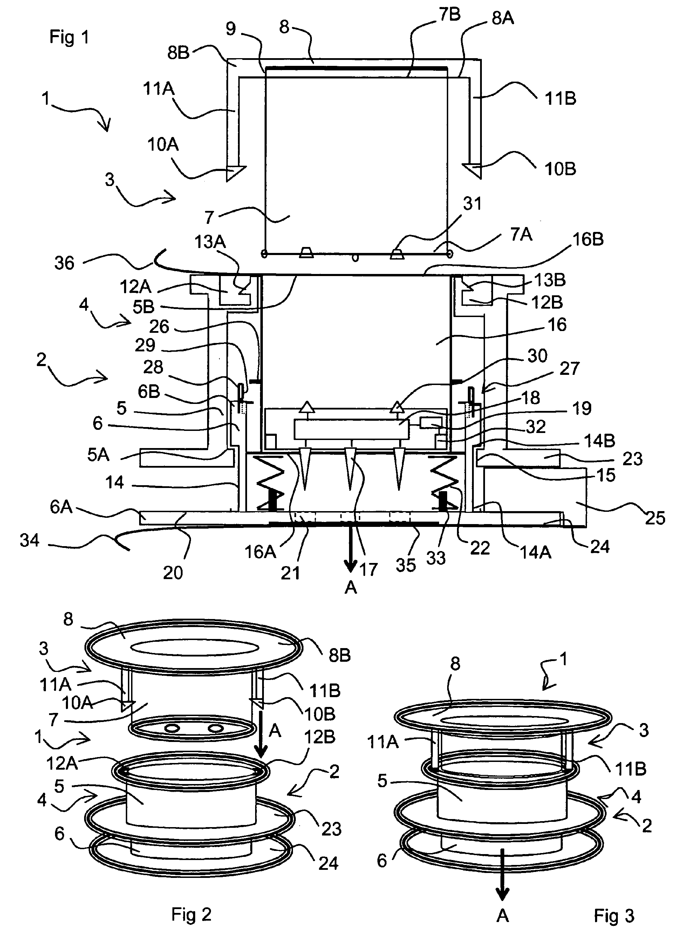

Sur la figure 1, on a représenté un exemple de réalisation du dispositif d'injection 1 selon l'invention qui comporte un boîtier 2 et une cartouche 3 insérée dans le boîtier 2. Le boîtier 2 définit une enceinte 4 allongée selon une certaine direction axiale indiquée par la flèche A. L'enceinte 4 est formée par deux tubes 5,6, ici cylindriques, insérés l'un dans l'autre de manière coaxiale selon la direction axiale A et agencés pour coulisser l'un dans l'autre dans les deux sens selon cette direction.

Comme visible sur la figure 1, le premier tube le plus extérieur 5 (tube fourreau) est ouvert à ses deux extrémités en 5A et 5B pour recevoir par le bas en 5A le deuxième tube 6 et par le haut en 5B la cartouche 3.

L'extrémité inférieure 6A du deuxième tube 6 forme la base du boîtier 2 destinée à être posée sur la peau d'un patient.

La cartouche 3 comprend un réservoir 7 tubulaire pré-rempli d'une substance médicamenteuse à injecter en 7B par un couvercle en forme de disque 8. Le couvercle 8 peut avoir une face intérieure 8A munie d'un évidemment 9 dans lequel s'emboîte le haut du réservoir 7. Le réservoir 7 a ici une forme cylindrique, de sorte à s'insérer de manière coaxiale selon la direction A sans trop de jeu dans les tubes 5,6. Selon l'invention, on peut avoir différentes cartouches avec des réservoirs 7 de volumes variés en verre ou en matériau plastique rigide mais tous adaptés pour s'insérer dans un même boîtier 2.

Comme on peut le voir figure 1, un support 16 en forme de panier cylindrique est fixé au tube 5 à sa partie supérieure 16B et coulisse dans le tube 6 selon la direction axiale A. Le fond 16A du support 16 porte une ou plusieurs aiguilles 17 s'étendant selon la direction axiale A et une pompe 18 destinée à faire circuler la substance médicamenteuse depuis le réservoir 7 vers la ou les aiguilles 17. La pompe 18 avec la ou les aiguilles 17 portées par le support 16 sont donc solidaires en translation avec le premier tube 5 dans le deuxième tube 6 selon la direction axiale A.

Selon l'invention, il est prévu une première partie femelle 13A,13B d'un système de fixation de la cartouche 3 dans l'enceinte 4 qui est destinée à s'accoupler de manière irréversible avec une deuxième partie mâle 10A,10B du système de fixation prévue sur la cartouche 3 lorsque la cartouche 3 est suffisamment insérée dans l'enceinte 4 selon la direction axiale A. Plus précisément, le couvercle 8 de la cartouche 3 comprend ici deux bras 11A, 11B diamétralement opposés s'étendant axialement à partir de la face intérieure 8A et dotés respectivement à leur extrémité libre de crans 10A, 10B formant la partie mâle du système de fixation. La partie femelle du système de fixation est constituée ici par des encoches 13A,13B disposées au fond de deux trous borgnes 12A,12B diamétralement opposés aménagées à l'intérieur de la paroi cylindrique du premier tube extérieur 5. Quand les crans 10A, 10B sont enclenchés dans les encoches 13A, 13B, il n'est plus possible de déverrouiller le système de fixation depuis l'extérieur de l'enceinte 4.

De manière avantageuse, les trous borgnes 12A,12B sont suffisamment profonds selon la direction axiale A pour servir de guidage axial pour l'insertion de la cartouche 3 dans l'enceinte 4, ce qui empêche toute rotation de la cartouche 3, lorsqu'elle est insérée dans l'enceinte 4.

On a représenté ici que deux bras 11A, 11B et avec deux crans 10A, 10B et que deux trous borgnes 12A,12B avec deux encoches 13A,13B, mais il va de soi qu'on pourrait concevoir un système de fixation avec un plus grand nombre de parties mâle et femelle.

Le tube 6 a une surface extérieure périphérique qui définit une gorge circonférentielle 14 dans laquelle vient coulisser de façon limitée dans la direction A une collerette annulaire intérieure du tube 5. La gorge 14 s'étend sur une certaine distance selon la direction axiale A entre deux épaulements d'extrémité 14A, 14B servant de butées pour la protubérance annulaire 15, cette distance correspondant à la course du tube 5 dans le tube 6. Cette course correspond aussi à la course des aiguilles 17 dans l'enceinte 4 et détermine la profondeur de pénétration des aiguilles 17 dans le derme du patient.

Les aiguilles ont un diamètre d'environ 0,05 mm à 0,2 mm pour une longueur d'environ 0,5 mm à 3 mm, selon les applications médicales. Elles sont fabriquées dans des matériaux adaptés pour usage médical, comme l'inox médical, des polymères ou des céramiques par exemple. Les aiguilles peuvent être réalisées de manière à pouvoir retenir en surface des produits désinfectants ou anesthésiants ou cicatrisants, par exemple en ayant une surface présentant des microrayures, des évents ou une certaine porosité.

Le dispositif d'injection 1 selon l'invention est donc conçu pour que les aiguilles 17 pénètrent à une profondeur prédéterminée dans le derme ou dans les tissus musculaires adaptée au traitement médical, en fonction du type d'injection hypodermique, intradermique, intramusculaire ou intraveineuse. Par exemple, dans le cas d'une injection sous cutanée, les aiguilles 17 pénètrent dans le derme jusqu'à une distance de 0,2 millimètre (mm) à 0,8 mm. Dans le cas d'une injection intramusculaire, les aiguilles 17 pénètrent dans les tissus musculaires jusqu'à une distance de 2 mm à 3 mm.

L'extrémité inférieure 6A du tube 6 est avantageusement fermée par une paroi 20 inférieure percée d'une ou plusieurs ouvertures 21 alignées axialement respectivement avec la ou les aiguilles 17. Des ressorts 22 sont interposés entre la paroi 20 et le support 16 et s'opposent au déplacement axial du support 16 en direction de la paroi 20.

Les tubes 5,6 sont bordés à leurs extrémités inférieures 5A,6A respectives de deux collerettes 23,24 annulaires coaxiales de diamètres identiques. Le couvercle en forme de disque 8 de la cartouche 3 forme aussi une collerette 8B de diamètre identique à celui des collerettes 23,24.

Comme on peut le voir sur la figure 1, les deux collerettes 23,24 peuvent être maintenues à distance l'une de l'autre par une entretoise sous la forme d'une bague de sécurité 25 empêchant le déplacement relatif en translation du tube 5 par rapport au tube 6. La bague 25 peut être prévue pour être arrachée ou présenter une zone sécable de façon à pourvoir être retirée du dispositif 1 par une opération simple de traction d'une seule main par exemple.

Le support 16 est muni d'un ou plusieurs cônes de percussion 30 pointant en direction d'opercules complémentaires 31 disposés sur la paroi inférieure 7A du réservoir 7. Les cônes de percussion 30 sont destinés à traverser les opercules 31 respectifs pour créer un passage entre le réservoir 7 et la pompe 18 lorsque la cartouche 3 est insérée dans l'enceinte 4 selon la direction axiale A. Plus particulièrement, les cônes de percussion 30 traversent les opercules 31 seulement après que les crans 10A, 10B soient enclenchés dans les encoches 13A,13B. De préférence, les cônes de percussion 30 sont entourés d'un joint (non montré) réalisant l'étanchéité de la connexion entre les cônes 30 et les opercules 31.

Le support 16 est par ailleurs équipé d'un interrupteur 32 dont la fonction est d'actionner la pompe 18 quand le tube 5 a été suffisamment déplacé dans le tube 6 selon la direction A. L'interrupteur 32 est actionné par un pion 33 disposé sur la paroi 20 à l'intérieur du tube 6 et qui vient traversé le support 16, ce qui commande alors le fonctionnement de la pompe 18.

La pompe 18 peut être une pompe à moteur électrique alimenté par une batterie 19 à travers l'interrupteur 32, la batterie 19 pouvant être logée dans le support 16. Dans l'exemple de réalisation de l'invention, la pompe 18 peut être réalisée à partir de microsystèmes mécaniques ou électromécaniques de microfluidique appelés MEMS ou MOEMS alimentés au moyen de la batterie 19 qui peut être une pile, une bio pile, un combustible interne ou un gaz inerte. L'autonomie de l'alimentation conditionne avantageusement le temps d'injection de la substance médicamenteuse. La pompe 18 du dispositif d'injection 1 peut par exemple être une pompe péristaltique, rotative ou centrifuge, ou une pompe à membranes ou à palettes, ou plus généralement de toute technologie adaptée permettant le pompage et l'injection.

Le support 16 est muni sur sa paroi extérieure d'une sorte de collerette 26 destinée à coopérer avec un moyen de blocage 27 à cliquet disposé sur l'extrémité supérieure 6B du tube intérieur 6. Le moyen de blocage 27 a pour fonction de laisser passer une seule fois dans un sens et dans l'autre le support 16 selon la direction axiale mais de l'empêcher de passer une seconde fois dans le même sens selon cette direction. Plus précisément, le moyen de blocage 27 comprend un support 28 et un levier 29 en forme de L. Le support 28 s'étend verticalement et est fixé à l'extrémité supérieure 6B du tube 6. Le support 28 est également équipé d'un pivot autour duquel tourne le levier 29. En particulier, le levier 29 pivote autour d'un axe perpendiculaire à la direction axiale A passant par l'intersection des branches de la forme en L. Dans la figure 1, le moyen de blocage 27 est montré dans un état avant blocage, dans lequel la branche verticale de la forme en L est située au dessus de la branche horizontale. La branche horizontale de la forme en L est alors située en dessous de la collerette 26 de le support 16, la branche horizontale et la collerette 26 étant agencées pour qu'un premier déplacement de le support 16 en translation vers le bas selon la direction axiale A entraîne par contact la rotation du levier 29. Un mouvement consécutif vers le haut du support 16 ne modifie pas la position du levier 29 qui bloque alors tout déplacement ultérieur en translation vers le bas du support 16. Ainsi, après utilisation, le dispositif d'injection 1 est bloqué dans une position dans laquelle les aiguilles 16 ne dépassent pas de l'enceinte 4, ne risquant donc pas de blesser ou contaminer l'utilisateur.

La taille du dispositif d'injection 1 varie entre 2 à 5 cm de diamètre et entre 2 à 5 cm en hauteur selon le produit à injecter. La taille de la pompe 18 varie entre 2 à 5 cm en largeur et longueur et entre 0,5 à 2 cm en hauteur selon le produit à injecter.

Initialement, l'enceinte 4 du dispositif d'injection 1 selon l'invention est stérile et conditionnée de manière à rester stérile jusqu'à utilisation du dispositif d'injection 1. En particulier, l'ouverture 5B du premier tube 5 recevant la cartouche 3 est aussi recouverte par un opercule servant de film de protection 36. La fonction de ce premier film de protection 36 est de conserver stérile l'intérieur du tube 5 et en particulier les cônes de percussion 30. Par ailleurs, la paroi 20 inférieure du tube 6, et en particulier les ouvertures 21, peut être recouverte par un opercule servant de film de protection 34. La fonction de ce deuxième film de protection 34 est de conserver stérile l'intérieur du tube 6 et en particulier les aiguilles 17. Avantageusement, le film de protection 34 recouvre et maintient stérile toute la surface de la paroi 20 destinée à être en contact avec la peau du patient, tant quand le dispositif n'est pas utilisé. On peut prévoir que le film de protection 34 et la bague de sécurité 25 soient fixés l'un avec l'autre de sorte que le retrait de l'un du dispositif d'injection 1 entraîne le retrait de l'autre. Entre la paroi 20 et le film de protection 34, une couche de feutre 35 peut être interposée de manière à recouvrir les ouvertures 21. Cette couche de feutre 35 est destinée à conserver stérile l'intérieur de la cavité une fois le film de protection 34 retiré. La couche de feutre 35 sera perforée par les aiguilles 17 lors de leur déplacement axial comme cela sera détaillé ci-dessous. De la même manière, la paroi inférieure 7A du réservoir 7, en particulier les opercules 31, peut être recouverte d'un film de protection (non montré) afin de maintenir stérile le contenu du réservoir 7. Enfin, la cartouche 3 peut être conditionnée dans une enveloppe protectrice stérile (non montrée).

Ainsi, avant utilisation, l'utilisateur a à sa disposition une ou plusieurs cartouches 3 stériles. Il peut donc choisir au dernier moment la substance médicamenteuse à injecter selon la pathologie du patient à traiter et insérer la cartouche 3 adéquate dans l'enceinte 4 du dispositif d'injection pour procéder à une injection comme décrit ci-dessous.

On décrit maintenant la succession des étapes de manipulation et de fonctionnement du dispositif d'injection 1 selon l'invention. L'utilisateur choisi une cartouche 3 contenant la substance qu'il désire injecter à un patient ou à lui-même. Il retire le premier film de protection 36 du premier tube 5 de l'enceinte 4 afin de pouvoir insérer la cartouche 3 dans l'enceinte 4, dans la direction indiquée par la flèche A sur la figure 1. Puis, il positionne les bras 11A, 11B de la cartouche dans les trous borgnes 12A,12B du boîtier et enfonce donc la cartouche 3 dans l'enceinte 4, les opercules 31 venant en face des cônes de percussion 30 correspondants (voir les figures 2 et 3).

Les crans 10A, 10B s'enclenchent dans les encoches 13A,13B, ce qui verrouille la cartouche 3 dans l'enceinte 4 de manière irréversible. En même temps, les cônes de percussions 30 viennent en contact avec les opercules 31 du réservoir 7 jusqu'à les percer de sorte à créer un passage étanche pour que la substance médicamenteuse contenue dans le réservoir 7 soit transmise vers la pompe 32. La pompe 18 n'étant pas en fonctionnement, la substance médicamenteuse est maintenue bloquée au niveau de la pompe.

L'utilisateur retire maintenant le deuxième film de protection 34 du deuxième tube 6 stérile et la bague de sécurité 25. Les aiguilles 17 sont alors complètement rentrées dans le deuxième tube 6 de l'enceinte 4 et l'utilisateur ne risque pas de se blesser, ni d'appréhender la piqûre.

L'utilisateur place la base 6A du boîtier contre le derme du patient à l'emplacement où l'injection doit être réalisée. Il procède ensuite à l'injection en exerçant une légère pression sur le couvercle 8 de la cartouche 3 dans le sens indiquée par la flèche A sur la figure 1. Cette pression déclenche deux phases successives de l'injection : la piqûre, puis l'injection de la substance à proprement dit. Un mouvement de translation axial est transmis par cette pression à l'ensemble formé par le premier tube 5, le support 16 avec la pompe 18 et la ou les aiguilles 17 et la cartouche 3 qui se déplace alors axialement en direction de l'extrémité inférieure 6A du deuxième tube 6. Plus particulièrement, les aiguilles 17 se déplacent en direction des ouvertures 21 dans la paroi inférieure 20 du deuxième tube 6 jusqu'à perforer la couche de feutre 35 stérile, puis pénétrer dans le derme ou le muscle du patient, tandis que les ressorts 22 se compriment. Le déplacement de l'ensemble formé par le premier tube 5, le support 16 et la cartouche 3, et par conséquent ici la pénétration des aiguilles 17 dans le derme, s'arrête lorsque la collerette 23 du tube 5 arrive en butée contre la collerette 24 du tube 6. Ce déplacement provoque la commutation du moyen de blocage 27 et simultanément la commutation de l'interrupteur 32. La pompe 18 se met en fonctionnement alors que les aiguilles 17 ont pénétré dans le derme ou le muscle à la profondeur nécessaire pour le traitement médical. Le temps d'injection varie en fonction du nombre d'aiguilles 17 et du volume de la substance médicamenteuse à injecter. Avantageusement, le temps d'injection est imposé par l'autonomie de la batterie 19 alimentant la pompe 18. Quand l'injection est terminée, l'utilisateur relâche la pression exercée sur le couvercle 8 de la cartouche 3 ce qui provoque la rétractation des aiguilles 17 dans le tube 6 dans le sens opposé à la flèche A, par la détente des ressorts 22 et le déclenchement du cliquet 29. Un nouveau déplacement axial des aiguilles 17 dans le deuxième tube 6 est donc empêché ce qui permet que le dispositif d'injection 1 peut être manipulé en toute sécurité car les aiguilles 17 ne sont plus mobiles hors de l'enceinte 4. En outre, le dispositif d'injection 1 n'est plus utilisable car le système de fixation 10A,10B,13A,13B de la cartouche 3 dans l'enceinte 4 est verrouillé de manière irréversible.

On comprend que la piqûre du derme du patient par les aiguilles 17 et l'injection de la substance médicamenteuse a été effectuée par une seule pression de l'utilisateur sur le dispositif d'injection, ce qui permet aisément à un utilisateur de pratiquer une auto-injection sans besoin d'être formé préalablement à un geste médical, ce qui peut être utile par exemple en cas de pandémie qui demande une forte réactivité d'urgence ou en cas de vaccination de masse.

## Revendications

1. Dispositif d'injection (1) miniaturisé à usage médical comprenant un boîtier (2) formant une enceinte(4) allongée selon une direction axiale (A) destinée à recevoir une cartouche (3) définissant un réservoir (7) pour une substance médicamenteuse à injecter, et comprenant en outre au moins une aiguille (17) et une pompe (18) mobiles en translation selon ladite direction axiale (A) dans ladite enceinte (4), ladite pompe (18) étant agencée pour faire circuler ladite substance médicamenteuse à injecter depuis ledit réservoir (7) vers ladite aiguille (17), **caractérisé en ce que** ladite aiguille (17) est destinée à être raccordée audit réservoir (7), avant utilisation, quand ladite cartouche (3) est insérée dans ladite enceinte (4) dudit boîtier (2), **en ce que** ledit dispositif d'injection (1) comprend en outre :
- une première partie (13A, 13B) d'un système de fixation du type mâle/femelle à crans de verrouillage pour verrouiller de manière irréversible ladite cartouche (3) dans ladite enceinte (4), ladite première partie (13A,13B) étant destinée à s'accoupler de manière irréversible par insertion selon ladite direction axiale (A) avec une deuxième partie (10A, 10B) dudit système de fixation prévue sur ladite cartouche (3), avant utilisation, quand ladite cartouche (3) est suffisamment insérée dans ladite enceinte (4) selon ladite direction axiale (A),
**en ce que** ladite pompe (18), ladite aiguille (17) et ladite cartouche (3) sont montées sur et solidaires d'un support (16) mobile en translation selon ladite direction axiale (A) dans ladite enceinte (4) pour réaliser la piqûre puis l'injection, **en ce que** ledit dispositif d'injection (1) comprend en outre :
- un moyen de sécurité (25) agencé pour, avant utilisation, empêcher le déplacement dudit support (16) mobile selon ladite direction axiale (A) lorsque ladite cartouche (3) est insérée dans l'enceinte (4),
- un moyen interrupteur (32) agencé pour être actionné, lors de l'utilisation, en réponse au déplacement selon ladite direction axiale (A) dudit support (16) mobile et commander alors le fonctionnement de ladite pompe (18).

2. Dispositif d'injection (1) selon la revendication 1, **caractérisé en ce que** ledit boîtier (2) comprend un premier et un deuxième tubes (5, 6), ledit premier tube (5) étant ouvert à ses deux extrémités (5A, 5B) pour recevoir par une extrémité (5A) ledit deuxième tube (6) et par l'autre extrémité ladite cartouche (3), l'extrémité (6A) dudit deuxième tube (6) formant la base dudit boîtier (2) destinée à être posée sur la peau d'un patient, ledit premier tube (5) étant apte à coulisser par rapport audit deuxième tube (6) selon ladite direction axiale (A), lesdits premier et deuxième tubes (5, 6) étant séparés par des ressorts (22) s'opposant au déplacement axial dudit premier tube (5) vers l'extrémité (6A) dudit deuxième tube (6).

3. Dispositif d'injection (1) selon la revendication 1, dans lequel les première et deuxième parties (13A,13B,10A,10B) dudit système de fixation sont agencées pour réaliser un guidage axial de ladite cartouche (3) dans ladite enceinte (4) quand ladite cartouche (3) est insérée dans ladite enceinte (4) selon ladite direction axiale (A).

4. Dispositif d'injection (1) selon la revendication 1, comprenant au moins un cône de percussion (30) qui est fixé audit support (16) et qui est raccordé à ladite pompe (18), les crans de verrouillage des première et seconde parties (13A, 13B) dudit système de fixation étant agencés pour se verrouiller quand ledit cône de percussion (30) pénètre dans ledit réservoir (7) de ladite cartouche (3).

5. Dispositif d'injection (1) selon la revendication 1, comprenant en outre un moyen de blocage (27) agencé pour laisser passer une seule fois dans un sens et dans l'autre ledit support (16) selon ladite direction axiale (A) et pour l'empêcher de passer une seconde fois dans le même sens selon cette direction axiale (A).

6. Dispositif d'injection (1) selon l'une des revendications précédentes, dans lequel une première partie femelle (13A, 13B) dudit système de fixation est prévue dans un trou borgne (12A,12B) aménagé à l'intérieur de ladite enceinte (4) dudit boîtier (2) et une deuxième partie mâle (10A, 10B) dudit système de fixation est prévue sur ladite cartouche (3) à une extrémité d'un bras (11A, 11B) s'étendant selon ladite direction axiale (A) et s'insérant dans ledit trou borgne (12A, 12B).

7. Dispositif d'injection (1) selon la revendication 6, dans lequel ladite deuxième partie mâle (10A, 10B) dudit système de fixation est prévue sur un couvercle (8) de ladite cartouche (3) à des extrémités respectives de deux bras (11A, 11B) diamétralement opposés par rapport audit couvercle (8) de ladite cartouche (3) et s'insérant dans deux trous borgnes (12A, 12B) diamétralement opposés aménagés à l'intérieur de ladite enceinte (4).

8. Dispositif d'injection (1) selon la revendication 2, dans lequel ledit moyen de sécurité (25) est une bague de sécurité maintenue entre deux collerettes (23,24) bordant respectivement ledit premier et ledit second tube (5, 6).

9. Dispositif d'injection (1) selon la revendication 2, dans lequel ledit moyen interrupteur (32) est monté sur ledit support (16) et qui est actionné par un pion (33) disposé sur ledit deuxième tube (6).

10. Dispositif d'injection (1) selon la revendication 7, dans lequel ledit couvercle (8) de ladite cartouche (3) est muni d'un évidement (9) dans lequel s'emboîte ledit réservoir (7).

11. Dispositif d'injection selon la revendication 1, dans lequel ledit moyen de sécurité est amovible de façon à pouvoir être retiré du dispositif d'injection.

## Claims

1. Miniaturized injection device (1) for medical use comprising a housing (2) forming an elongated chamber (4) in an axial direction (A) designed to receive a cartridge (3) defining a reservoir (7) for a drug substance to be injected, and further comprising at least a needle (17) and pump (18) both movable in translation following said axial direction (A) in said chamber (4), said pump (18) being arranged for flowing said drug substance to be injected from said reservoir (7) toward said needle (17), **characterized in that** said needle (17) is designed to be connected to said reservoir (7), before use, when said cartridge (3) is inserted in said chamber (4) of said housing (2), and **in that** said injection device (1) further comprising:
- a first part (13A, 13B) male/female type of a fixing system with locking notches to irreversibly lock said cartridge (3) in said chamber (4), said first part (13A, 13B) being designed to be irreversibly joined by insertion following said axial direction (A) with a second part (10A, 10B) of said fixture system provided on said cartridge (3), before use, when said cartridge (3) is inserted sufficiently following said chamber (4) in the axial direction (A),
**in that** said pump (18), said needle (17) and said cartridge (3) are assembled and fixed in translation following the axial direction (A) with a movable support (16) in said chamber (4) to perform the insertion and then the injection, and **in that** said injection device (1) further comprises:
- a safety means (25) designed to, before use, displacement the movement of said movable support (16) following the axial direction (A) when said cartridge (3) is inserted in the chamber (4),
- a switching means (32) designed to be activated, during use, in response of the displacement of said movable support (16) following the axial direction so as to control said pump (18) operation.

2. Injection device (1) according to claim 1, **characterized in that** said housing (2) comprises a first and a second tubes (5, 6) said first tube (5) being opened at both ends (5A, 5B) for receiving by one end (5A) said second tube (6) and by the other end said cartridge (3), the end (6A) of said second tube (6) forming the basis of said housing (2) designed to be placed on patient skin, said first tube (5) being suitable to slide with respect of said second tube (6) following the axial direction (A), said first and second tubes (5, 6) being separated by springs (22) opposing to the axial movement of said first tube (5) toward the end (6A) of said second tube (6).

3. Injection device (1) according to claim 1, wherein first and second parts (13A, 13B, 10A, 10B) of said fixing system are designed for performing an axial guide of said cartridge (3) in said chamber (4) when said cartridge (3) is inserted in said chamber (4) following said axial direction (A).

4. Injection device (1) according to claim 1, comprising at least an impacting cone (30) which is fixed on said support (16) and which is connected to said pump (18), locking notches of first and second parts (13A, 13B) of said fixing system being designed to lock when said impacting cone (30) penetrates in said reservoir (7) of said cartridge (3).

5. Injection device (1) according to claim 1, further comprising a blocking means (27) designed to permit said support (16) to pass following the axial direction (A) only once in one direction and in the other direction and to prevent it to pass following said axial direction (A) twice in same direction.

6. Injection device (1) according to one of the preceding claims, wherein a first female part (13A, 13B) of said fixing system is provided in a one-eyed hole (12A, 12B) provided inside said chamber (4) of said housing (2) and a second male part (10A, 10B) of said fixing system is provided on said cartridge (3) at one end of an arm (11A, 11B) extending following the axial direction (A) and inserting in said one-eyed hole (12A, 12B).

7. Injection device (1) according to claim 6, wherein said second male part (10A, 10B) of said fixing system is provided on a lid (8) of said cartridge (3) at respective ends of both arms (11A, 11B) diametrically opposite relative to said lid (8) of said cartridge (3) and inserting in two one-eyed holes (12A, 12B) diametrically opposite provided inside said chamber (4).

8. Injection device (1) according to claim 2, wherein said safety means (25) is a safety ring maintained between two collars (23, 24) respectively bordering said first and second tube (5, 6).

9. Injection device (1) according to claim 2, wherein said switching means (32) is assembled said support (16) and which is activated by a pin (33) provided on said second tube (6).

10. Injection device (1) according to claim 7, wherein said lid (8) of said cartridge (3) is provided with a recess (9) in which said reservoir (7) fits in.

11. Injection device (1) according to claim 1, wherein said safety means is removable so as to be removed from the injection device.

## Patentansprüche

1. Miniaturisierte Injektionsvorrichtung (1) zum medizinischen Einsatz, umfassend ein Gehäuse (2), das einen gemäß einer axialen Richtung (A) langgestreckten Bereich (4) bildet, der dazu bestimmt ist, eine Kartusche (3) aufzunehmen, die ein Reservoir (7) für eine zu injizierende medikamentöse Substanz definiert, und umfassend ferner wenigstens eine Nadel (17) und eine Pumpe (18), die translatorisch gemäß der axialen Richtung (A) in dem Bereich (4) beweglich sind, wobei die Pumpe (18) ausgebildet ist, um die zu injizierende medikamentöse Substanz von dem Reservoir (7) zu der Nadel (17) zu bewegen, **dadurch gekennzeichnet,**
**dass** die Nadel (17) dazu bestimmt ist, an das Reservoir (7) angeschlossen zu werden, vor dem Einsatz, wenn die Kartusche (3) in den Bereich (4) des Gehäuses (2) eingeführt ist,
**dass** die Injektionsvorrichtung (1) ferner umfasst:
- ein erstes Teil (13A, 13B) eines Fixiersystems vom männlichen/weiblichen Typ mit Verriegelungsrasten zum irreversiblen Verriegeln der Kartusche (3) in dem Bereich (4), wobei das erste Teil (13A, 13B) dazu bestimmt ist, sich irreversibel durch Einführen gemäß der axialen Richtung (A) mit einem an der Kartusche (3) vorgesehenen zweiten Teil (10A, 10B) des Fixiersystems zu verbinden, vor dem Einsatz, wenn die Kartusche (3) ausreichend in den Bereich (4) gemäß der axialen Richtung (A) eingeführt ist,
**dass** die Pumpe (18), die Nadel (17) und die Kartusche (3) auf einem in dem Bereich (4) gemäß der axialen Richtung (A) translatorisch beweglichen Support (16) montiert und an diesem festgelegt sind, um den Stich und anschließend die Injektion zu realisieren,
**dass** die Injektionsvorrichtung (1) ferner umfasst:
- ein Sicherungsmittel (25), das ausgebildet ist, um, vor dem Einsatz, die Verlagerung des gemäß der axialen Richtung (A) beweglichen Supports (16) zu verhindern, wenn die Kartusche (3) in den Bereich (4) eingeführt ist,
- ein Unterbrechungsmittel (32), das ausgebildet ist, um, anlässlich des Einsatzes, in Reaktion auf die Verlagerung des beweglichen Supports (16) gemäß der axialen Richtung (A) betätigt zu werden und den Betrieb der Pumpe (18) zu steuern.

2. Injektionsvorrichtung (1) nach Anspruch 1, **dadurch gekennzeichnet, dass** das Gehäuse (2) einen ersten und einen zweiten Tubus (5, 6) umfasst, wobei der erste Tubus (5) an seinen beiden Enden (5A, 5B) offen ist, um durch ein Ende (5A) den zweiten Tubus (6) und durch das andere Ende die Kartusche (3) aufzunehmen, wobei das die Basis des Gehäuses (2) bildende Ende (6A) des zweiten Tubus (6) dazu bestimmt ist, auf die Haut eines Patienten aufgelegt zu werden, wobei der erste Tubus (5) ausgebildet ist zum Verlagern in Bezug auf den zweiten Tubus (6) gemäß der axialen Richung (A), wobei der erste Tubus und der zweite Tubus (5, 6) durch Federn (22) voneinander fort gehalten sind, die der axialen Verlagerung des ersten Tubus (5) zu dem Ende (6A) des zweiten Tubus (6) entgegenwirken.

3. Injektionsvorrichtung (1) nach Anspruch 1, bei der die ersten und zweiten Teile (13A, 13B, 10A, 10B) des Fixiersystems angeordnet sind zum Realisieren einer axialen Führung der Kartusche (3) in dem Bereich (4), wenn die Kartusche (3) in den Bereich (4) gemäß der axialen Richtung (A) eingeführt wird.

4. Injektionsvorrichtung (1) nach Anspruch 1, umfassend wenigstens einen Stoßkonus (30), der an dem Support (16) fixiert ist und der an die Pumpe (18) angeschlossen ist, wobei die Verriegelungsrasten der ersten und zweiten Teile (13A, 13B) des Fixiersystems ausgebildet sind, um sich zu verriegeln, wenn der Stoßkonus (30) in das Reservoir (7) der Kartusche (3) eindringt.

5. Injektionsvorrichtung (1) nach Anspruch 1, umfassend ferner ein Blockiermittel (27), das ausgebildet ist zum einmaligen Passierenlassen des Supports (16) in eine und der entgegengesetzten Ausrichtung gemäß der axialen Richtung (A) und zum Verhindern von dessen zweiten Passieren in der selben Ausrichtung gemäß der axialen Richtung (A).

6. Injektionsvorrichtung (1) nach einem der vorhergehenden Ansprüche, bei der ein erstes weibliches Teil (13A, 13B) des Fixiersystems in einem Sackloch (12A, 12B) vorgesehen ist, das innerhalb des Bereichs (4) des Gehäuses (2) ausgebildet ist, und bei der ein zweites männliches Teil (10A, 10B) des Fixiersystems an der Kartusche (3) an einem Ende eines Zapfens (11A, 11 B), der sich gemäß der axialen Richtung (A) erstreckt und in das Sackloch (12A, 12B) einführbar ist, vorgesehen ist.

7. Injektionsvorrichtung (1) nach Anspruch 6, bei der das zweite männliche Teil (10A, 10B) des Fixiersystems an einem Deckel (8) der Kartusche (3) an entsprechenden Enden von zwei in Bezug auf den Deckel (8) der Kartusche (3) diametral entgegengesetzten Zapfen (11A, 11B) vorgesehen ist, die in zwei im Inneren des Bereichs (4) diametral entgegengesetzt ausgebildete Sacklöcher (12A, 12B) einführbar sind.

8. Injektionsvorrichtung (1) nach Anspruch 2, bei der das Sicherungsmittel (25) ein Sicherungsring ist, der zwischen zwei den jeweils ersten und zweiten Tubus (5, 6) begrenzenden Flanschen (23, 24) gehalten ist.

9. Injektionsvorrichtung (1) nach Anspruch 2, bei der das Unterbrechungsmittel (32) an dem Support (16) angebracht ist und durch einen an dem zweiten Tubus (6) angeordneten Stift (33) betätigt wird.

10. Injektionsvorrichtung (1) nach Anspruch 7, bei der der Deckel (8) der Kartusche (3) mit einer Ausnehmung (9) versehen ist, in welche das Reservoir (7) eingepasst werden kann.

11. Injektionsvorrichtung nach Anspruch 1, bei der das Sicherungsmittel abnehmbar ist, um von der Injektionsvorrichtung zurückgezogen werden zu können.
